# EUROPÄISCHE PATENTSCHRIFT

(11) **EP 1 913 139 B1**
(45) Veröffentlichungstag und Bekanntmachung des Hinweises auf die Patenterteilung: **19.08.2009**
(21) Anmeldenummer: 06763002.0
(22) Anmeldetag: 07.08.2006
(51) Int. Cl.: C12N 15/10, C40B 10/00

(54) **VERFAHREN FÜR DIE KONTINUIERLICHE ZIELGERICHTETE EVOLUTION VON PROTEINEN IN VIVO**
METHODS FOR CARRYING OUT THE SELECTIVE EVOLUTION OF PROTEINS IN VIVO
PROCÉDÉS POUR L'ÉVOLUTION CIBLÉE CONTINUE DE PROTÉINES IN VIVO

(30) Priorität: 08.08.2005 DE 102005037349
(43) Veröffentlichungstag der Anmeldung: 23.04.2008
(73) Patentinhaber: GENEART AG, 93053 Regensburg (DE)
(72) Erfinder: LISS, Michael, 93047 Regensburg (DE)
(74) Vertreter: Weiss, Wolfgang
(86) Internationale Anmeldenummer: PCT/EP2006/007797
(87) Internationale Veröffentlichungsnummer: WO 2007/017228

(56) Entgegenhaltungen:
- WO-A-20/04108926
- WO-A2-02/22869
- WO-A2-02/083892
- LI B ET AL: "Identification of mutations in p53 that affect its binding to SV40 large T antigen by using the yeast two-hybrid system." THE FASEB JOURNAL : OFFICIAL PUBLICATION OF THE FEDERATION OF AMERICAN SOCIETIES FOR EXPERIMENTAL BIOLOGY. JUL 1993, Bd. 7, Nr. 10, Juli 1993 (1993-07), Seiten 957-963, XP001002694 ISSN: 0892-6638
- SUZUKI M ET AL: "RANDOM MUTAGENESIS OF THERMUS AQUATICUS DNA POLYMERASE I: CONCORDANCE OF IMMUTABLE SITES IN VIVO WITH THE CRYSTAL STRUCTURE" PROCEEDINGS OF THE NATIONAL ACADEMY OF SCIENCES OF USA, NATIONAL ACADEMY OF SCIENCE, WASHINGTON, DC, US, Bd. 93, September 1996 (1996-09), Seiten 9670-9675, XP002062865 ISSN: 0027-8424

## Beschreibung

Die vorliegende Erfindung betrifft Verfahren zur Herstellung von Varianten von Proteinen, welche im Vergleich mit dem Ausgangsprotein verbesserte Eigenschaften aufweisen, wobei die Varianten mit Hilfe eines *in vivo* Evolutionsverfahrens erhalten werden.

### Hintergrund der Erfindung

Die zunehmende Bedeutung der Biotechnologie in den Bereichen der Medizin, der chemischen Industrie und der Agrarwirtschaft verlangt zunehmend nach Proteinen, die für ihren jeweiligen Einsatzzweck optimal angepasst sind. Zunächst werden diese Proteine hauptsächlich aus der Umwelt, meist im Rahmen von sog. Metagenom-Screenings isoliert. Vermehrt werden sie in der Folge über unterschiedliche Methoden an die geplanten "artifiziellen" Einsatzbedingungen angepasst.

So werden beispielsweise Enzyme benötigt, die hitzestabiler sind als ihre natürlichen Varianten, eine andere Substratspezifität haben oder höhere Aktivitäten zeigen. Pharmazeutische Proteine etwa sollen längere Halbwertszeiten aufweisen, um geringer dosiert werden zu können, oder über die hochaffine und spezifische Bindung an Zielmoleküle krankheitsassoziierte Stoffwechsel- oder Infektionswege inhibieren.

### Stand der Technik

Diese Anpassung geschieht zum Teil über rationale Proteindesign-Ansätze. Bislang gibt es jedoch nur sehr beschränkte Möglichkeiten, von der gewünschten Funktion - dem Phänotyp - eines Proteins auf dessen Struktur zu schließen, bzw. von der dreidimensionalen Struktur des Proteins die entsprechende Primärsequenz abzuleiten. Um dennoch eine Funktionssteigerung eines Proteins zu erreichen, bedient man sich derzeit teilevolutionärer Ansätze, die unter dem Begriff der "zielgerichteten Evolution" (Directed Evolution) zusammengefasst werden (Buskirk et al., 2003, "In vivo evolution of an RNA-based transcriptional activator". Chem. Biol. 10:533-540; de Crecy-Lagard et al., 2001, "Long term adaptation of a microbial population to a permanent metabolic constraint: overcoming thymineless death by experimental evolution of Escherichia coli", BMC Biotechnology 1:10; Fields and Song, 1989, "A novel genetic system to detect protein-protein interactions", Nature, 340: 245-246; Long-McGie et al., 1999, "Rapid in vivo evolution of a β-lactamase using phagemids", Biotechnol. Bioeng. 68:121-125; Rohde et al., 1995, "The mutant distribution of an RNA species replicated by Q beta replicase", J. Mol. Biol. 249:754-762).

Nach dem derzeitigen Stand der Technik beruhen die bisherigen Verfahren der Directed Evolution im Wesentlichen auf der Generierung einer Vielzahl von Varianten (Nachkommen) des zu verbessernden Proteins und deren Selektion nach verbesserten Abkömmlingen. Dabei kann die Zahl der untersuchten Mutanten z.T. sehr groß sein, liegt jedoch in der Regel bei unter 10¹¹. Betrachtet man ein Protein von nur 100 Aminosäuren, so existieren theoretisch 20¹⁰⁰ = 10¹³⁰ unterschiedliche Varianten davon. Eine Library (Bibliothek) einer Größe von 10¹¹ deckt demnach nur einen sehr kleinen Bruchteil der möglichen Varianten ab. Die Wahrscheinlichkeit, in einer solchen Library die theoretisch beste Variante zu finden, ist annähernd Null.

Das Prinzip der Evolution mit seinen drei Voraussetzungen - Replikation, Mutation und Selektion - vermag innerhalb eines gegebenen Systems eine gerichtete Entwicklung von einfachen bis hin zu hoch angepassten Strukturen zu bewirken. Dieses Modell des so genannten "blinden Uhrmachers" ermöglicht die Schaffung von Komplexität ohne ein planerisches Zutun und ohne notwendige Kenntnis von Strukturdaten.

Um eine große Anzahl von Varianten auf maximale Affinität zu einem Zielmolekül hin zu screenen, wurde bereits eine Vielzahl von Protokollen entwickelt (z.B. Yeast Two-Hybrid, Bacterial Display, Phage Display, Ribosomal Display, mRNA Display). Für das Screenen anderer Eigenschaften, wie etwa enzymatische Aktivität werden meist Assay-Formate benötigt, die die Untersuchung von nur einer verhältnismäßig kleinen Anzahl von Varianten (<10⁶) erlauben.

Diesen Methoden gemeinsam ist, dass sie auf die Generierung einer Library (= Mutation) sowie deren anschließende Selektion beschränkt sind. Eine Replikation (= nächste Generation von Mutanten des "Gewinners" der zuletzt erfolgten Selektion) ist mit diesen Protokollen zwar manuell möglich, jedoch ebenso aufwändig wie der vorangegangene Schritt. Daher erstrecken sich entsprechende Ansätze in aller Regel nur über ein bis zwei Generationen. Aus diesem Grund handelt es sich bei den erwähnten Protokollen nicht um evolutionäre Ansätze im eigentlichen Sinne, sondern vielmehr um die ausschließliche Selektion vorhandener Variantenpools.

Folglich kann das Potenzial der schrittweisen Adaption über viele Generationen hinweg nicht genutzt werden, was jedoch die notwendige Voraussetzung dafür wäre, unter Umständen selbst die aktivste Variante aus einer astronomischen Anzahl von Möglichkeiten zu identifizieren.

WO 2004/108926 offenbart Verfahren zum Evolvieren von Nukleinsäuren und Proteinen. Dieses Verfahren beruht auf der Ausnutzung der hohen Mutationsrate und Adaptationsfähigkeit von RNA-Viren, insbesondere des Bakteriophagen Phi. Ein β-Lactamase-Gen wurde in das Genom des Bakteriophagen Phi6 unter Bildung eines rekombinanten Phagen eingefügt. Dieser rekombinante Phi6-Phage wurde in Bakterienzellen unter Vermeidung der Lyse vermehrt, wobei durch Zugabe von Ampicillin und einem weiteren Antibiotikum Selektionsdruck ausgeübt wurde. Es wurde festgestellt, dass die überlebenden Bakterienzellen Phagen-RNA-Moleküle enthielten, welche ein gegenüber dem ursprünglich eingebrachten β-Lactamase-Gen auf Nukleinsäureebene verändertes β-Lactamase-Gen enthielten. Der Selektionsdruck hatte somit zu einer Evolution des β-Lactamase-Gens gerührt. Ebenfalls erwähnt sind weitere Möglichkeiten der Evolution von z.B. regulatorischen Proteinen oder RNAs oder Molekülen mit spezifischen Bindeeigenschaften. Nicht offenbart ist jedoch die Möglichkeit der Evolution von beliebigen Proteinen, welche in Bezug auf ihre Funktion vollkommen unabhängig sind von der Expression des jeweiligen Sektionsgens. Des Weiteren sieht die WO 2004/108926 einen separaten Selektions-/Screening-Schritt vor. Insbesondere die Offenbarung bezüglich der Evolution von Molekülen mit bestimmten Bindeeigenschaften erfordert nach jeder Generation der Evolution ein Screening, so dass das offenbarte System nicht vollkommen sich selbst überlassen werden kann.

Li B. et al. beschreiben mit Hilfe des Two-Hybrid-Systems eine Methode zur Identifizierung von p53 Mutationen, die die Bindung an das SV40 grosse T-Antigen modulieren. Das murine p53-Gen wurde mittels fehlerhafter PCR mutiert und in Hefezellen selektioniert, wobei abnehmende Reportergenaktivitäten auf fehlerhafte Wechselwirkungen zwischen den Tumorsupressor p53-Mutanten und dem grossen T-Antigen schliessen lassen (FASEB J. 7:957-963, 1993).
WO0222869 beschreibt, mittels NAP-Teilenzymen, die Selektion eines Paares von Polypeptiden mit bestimmten Bindeeigenschaften zueinander, wobei eine erste Nucleinsäure mit einer ersten NAP-Teilenzym kodierenden Nucleinsäure fusioniert und eine zweite Nucleinsäure mit einer zweiten NAP-Teilenzym kodierenden Nucleinsäure fusioniert wird. Die stabile Wechselwirkung erster und zweiter NAP-Teilenzym Proteindomänen führt, in Abhängigkeit von der Interaktion beider ersten und zweiten Nucleinsäuren, zu einem aktiven NAP-Enzym, dessen Aktivität analysiert werden kann.

Die Aufgabe der vorliegenden Erfindung war es daher, ein System zu schaffen, welches ein *in vivo*-Evolutionsverfahren bereitstellt, das auf aufwändige Screening-Methoden nicht angewiesen ist.

Diese Aufgabe wird gelöst durch ein Verfahren zur Herstellung von Varianten Y' eines Proteins Y, wobei die Variante Y' des Proteins Y durch veränderte Bindungseigenschaften an ein Targetmolekül X gekennzeichnet sind, umfassend die Schritte:
(a) Bereitstellen einer Zelle, enthaltend
   (a1) eine erste Nukleinsäure, die für ein zu variierendes Protein Y kodiert,
   (a2) eine zweite Nukleinsäure, die für das Targetmolekül X kodiert, und
   (a3) und eine dritte Nukleinsäure, welche für einen Evolutionsmarker unter der Kontrolle einer modulierbaren Expressionskontrollsequenz kodiert,
   wobei die Expression des Evolutionsmarkers durch die Bindung von Y und/oder Y' an X moduliert wird,
(b) Kultivieren der Zelle unter Bedingungen, so dass
   (b1) eine Bildung von Varianten Y' des Proteins Y erfolgt und
   (b2) eine Selektion auf diejenigen Zellen erfolgt, welche eine gegenüber der Zelle aus (a) modulierte Expression des Evolutionsmarkers aufweisen, und
(c) Identifizieren und gegebenenfalls Isolieren derjenigen Zellen, die eine modulierte Expression des Evolutionsmarkers aufweisen und
(d) gegebenenfalls Identifizieren und/oder Charakterisieren von Varianten Y' des Proteins Y in den Zellen aus (c).

Inhalt dieser Erfindung sind autonome Systeme, die einerseits die Selektion einer gegebenen Variantenbibliothek im Labor zulassen, und gleichzeitig einen Replikationsmechanismus beinhalten. Dabei soll die Selektion einzig - und wie bei der natürlichen Evolution - über eine bevorzugte Replikation besser angepasster Varianten realisiert werden. Des Weiteren ist das verwendete Replikationssystem so aufgebaut, dass es eine ausreichende Mutationsrate während der Replikationen erlaubt. Die Anzahl der auf diese Weise theoretisch getesteten Varianten eines Ausgangskonstrukts errechnet sich aus der Anzahl der Nachkommen eines Gewinners einer Generation zur Potenz der Gesamtanzahl der Generationen eines Experiments (z.B. 10 Nachkommen pro Generation bei 400 Generationen = 10⁴⁰⁰ mögliche Varianten.) Zwar ist es unmöglich, dass jede dieser 10⁴⁰⁰ einzelnen Varianten explizit in einem Experiment physikalisch vorhanden ist, jedoch sucht sich das System selbst einen "Pfad" innerhalb eines komplexen virtuellen Terrains, der stets hinauf bis zum absoluten Maximum führt. Nur die Varianten entlang des Pfades haben während des Experiments existiert, theoretisch möglich sind alle Punkte (Varianten) des Terrains.

Das erfindungsgemäße Verfahren beruht somit auf dem Prinzip, dass ein zu evolvierendes Protein Y, welches für die Zwecke der vorliegenden Erfindung als "zu variierendes Protein Y" bezeichnet wird, von einer Nukleinsäure kodiert wird, welche bei Expression *in vivo* zu Varianten Y' dieses Proteins führt, wobei die Bildung von Varianten auf Nukleinsäureebene erfolgt, z.B. durch Vervielfältigung (Replikation) der Nukleinsäure, die für Y kodiert, durch Polymerasen mit einer bestimmten Fehlerrate (z.B. weil sie keine Proofreading-Funktion haben).

Diese Varianten Y' können dann auf verschiedene Eigenschaften selektioniert werden, wie z.B. auf die Eigenschaft, an ein bestimmtes weiteres Protein mit höherer Affinität zu binden. Wenn diese höher affinen Bindungseigenschaften der Varianten Y' des Proteins Y für eine Aktivierung und/oder Verstärkung der Expression eines Evolutionsmarkergens geeignet sind, so kann bei Anwendung von Selektionsdruck autonom eine Evolution stattfinden, da nur diejenigen Zellen überleben bzw. sich vermehren bzw. sich schneller vermehren, welche Varianten des Proteins Y mit den gewünschten verbesserten Eigenschaften exprimieren.

Für ein derartiges System sind im Prinzip jede Art von Zellen geeignet, es können prokaryontische Zellen oder eukaryontische Zellen sein. Die Art der Zelle hängt gegebenenfalls von dem zu wählenden Selektionssystem ab, wobei der Fachmann in der Lage ist, die für das jeweilige Selektionssystem geeigneten Zellen auszuwählen. Es können Bakterienzellen, Hefezellen, Pflanzenzellen und Vertebratenzellen, insbesondere Säugerzellen, vorzugsweise humane Zellen, verwendet werden. Die Auswahl der Zelle hängt von den jeweiligen Evolutionssystem ab und kann vom Fachmann selbst ausgewählt werden.

Wie oben bereits erwähnt, umfasst das erfindungsgemäße Verfahren zwei Gruppen von Vorgängen, nämlich einerseits die Replikation und Mutation und zum anderen die Selektion. Für beide dieser Gruppen von Vorgängen sind bereits im Stand der Technik Verfahren und Systeme bekannt.

Um ein zu variierendes Protein Y hin zu Varianten Y' mit verbesserten Eigenschaften zu evolvieren, ist es zunächst notwendig, dass in einer *in vivo*-Umgebung, d.h. z.B. in einer Zelle, die Möglichkeit der Entstehung solcher Varianten bereitgestellt wird. Dies kann beispielsweise auf Nukleinsäureebene geschehen. Zum Beispiel können DNA-Polymerasen oder RNA-Polymerasen eingesetzt werden, welche eine gewisse Fehlerrate aufweisen, so dass bei der Vervielfältigung und/oder bei der Transkription der Nukleinsäure, welche für das Protein Y Kodiert, mutierte Nukleinsäuren entstehen, welches dann, z.B. aufgrund von Punktmutationen, Deletionen oder Insertionen, auf Proteinebene zu Varianten Y' des zu variierenden Proteins Y führen.

Die für die Replikation und Mutation verantwortlichen Polymerasen, d.h. DNA-abhängige und RNA-abhängige DNA- und RNA-Polymerasen können, je nach der gewählten Ausführungsform des erfindungsgemäßen Verfahrens, entweder bereits in der Zelle vorhanden sein oder sie können auch separat bereitgestellt werden, beispielsweise können sie von einer weiteren Nukleinsäure, z.B. einem Plasmid, kodiert werden.

Die erste Nukleinsäure, welche für das zu variierende Protein Y kodiert, liegt erfindungsgemäß in einer Form vor, die auf Nukleinsäureebene von Polymerasen in der Zelle repliziert werden kann. In einer bevorzugten Ausführungsform liegt die erste Nukleinsäure in Form eines RNA-Replikons vor.

Für die Zwecke der vorliegenden Erfindung bedeutet "Replikon" insbesondere eine Nukleinsäure, die von einer RNA-abhängigen RNA-Polymerase, nämlich einer so genannten Replikase, repliziert wird. Ein Replikon kann somit entweder eine RNA sein oder aber ein Replikon kann eine DNA sein, welche nach einem ersten Transkriptionszyklus für ein RNA-Replikon kodiert. Das RNA-Replikon ist vorzugsweise ausgewählt aus linearen RNA-Sequenzen, Genomen aus RNA-basierenden Organismen, RNA-Plasmiden, RNA-Viren, wie z.B. RNA-Bakteriophagen, und RNA-Analoga. Die erfindungsgemäß geeigneten Replikasen sind vorzugsweise, ausgewählt aus RNA-abhängigen RNA-Polymerasen, wie etwa Qβ-, Phi6-, Phi8-, Phi9-, Phi10-, Phi11,- Phi13- und Phi14-Replikasen, und ähnlichen (siehe z.B. WO 2004/108926 sowie die unten genannten Publikationen von Makaev et al.).

Replikasen sind weitaus ungenauer als DNA-Polymerasen, da sie keine Proofreading-Funktion besitzen. Ihre Fehlerrate liegt bei einer Mutation pro 10³ bis 10⁴ synthetisierte Basen. Zusätzlich zu der hohen Fehlerrate sind jedoch Replikasen, insbesondere die Qβ-Replikase, in hohem Maße substratspezifisch und können somit auf spezifische Ziel-RNAs "geeicht werden". Im Stand der Technik bekannte Replikasen sind z.B. die Qβ-Replikase oder Phi-Replikasen. Phi-Replikasen und deren Evolutionspotenzial sind bereits im Stand der Technik beschrieben worden, insbesondere von Makajev et al. in Journal of Virology, 2004, Band 78, Nr. 4, Seite 2114-2120, EMBO Journal, 2000, Band 19, Nr. 1, Seiten 124-133 und Virus Research, 2004, 101, 45-55 sowie in der Internationalen Patentanmeldung WO 2004/108926. Die genannte Patentanmeldung beschreibt ein System, bei dem als Replikon ein modifiziertes Genom des Bakteriophagen Phi6 und als Replikase die virale Phi6-Replikase verwendet wird. Das dort beschriebene System wird in E.coli-Zellen durchgeführt. Ein derartiges System ist auch für die vorliegende Erfindung geeignet.

Die zweite Nukleinsäure kodiert für ein Targetmolekül. Das Targetmolekül X ist vorzugsweise ein Protein, kann aber auch ein anderes durch Expression der zweiten Nukleinsäure erhaltenes Genprodukt sein, wie etwa eine mRNA oder deren Nukleinsäurederivate. Das Targetmolekül X wird für die Zwecke der vorliegenden Erfindung so gewählt, dass das Protein Y bzw. dessen Varianten Y' an dieses Targetmolekül X binden und eine Bindung von X und Y bzw. X und Y' die Expression des Evolutionsmarkers modulieren können. Die Nukleinsäure, die für das Targetmolekül X kodiert, kann in jeder geeigneten Form bereitgestellt werden, z.B. in Form eines Plasmids, welches für die Transfektion oder Transformation von Zellen geeignet ist. Dieses Plasmid ist somit vorzugsweise mit einem Selektionsmarker ausgestattet. Das Targetmolekül X kann für die Zwecke der vorliegenden Erfindung vollkommen frei gewählt werden, es kann somit jedes Zielmolekül, welches als Bindepartner für das gesuchte, zu variierende Protein Y fungiert, ausgewählt werden.

Um die erfindungsgemäße, zielgerichtete Evolution durchzuführen, muss auf das ausgewählte Zellsystem ein gewisser Selektionsdruck ausgeübt werden.

Aus diesem Grund kodiert die dritte Nukleinsäure für einen Evolutionsmarker unter der Kontrolle einer modulierbaren Expressionskontrollsequenz, wobei die Expression des Evolutionsmarkers durch die Bindung von dem Protein Y bzw. dessen Varianten Y' an ein Targetmolekül X moduliert wird. Die dritte Nukleinsäure wird vorzugsweise in Form eines Plasmids bereitgestellt, kann jedoch in jeder Form vorliegen, die sich für das Einbringen in eine Zelle eignet. Die modulierbare Expressionskontrollsequenz umfasst vorzugsweise zumindest einen Promotor und besonders bevorzugt weitere Sequenzen, z.B. Enhancer und dgl., insbesondere Upstream-Aktivierungssequenzen, wie z.B Aktivierungssequenzen auf der DNA, die die Bindung von Transkriptionsmodulatoren, insbesondere die Bindung von Transkriptionsaktivatoren, erlauben, wobei die Bindung jener Transkriptionsmodulatoren einen Einfluss auf die Expression des entsprechenden Gens, welches von der modulierbaren Expressionskontrollsequenz gesteuert wird, haben.

Der Evolutionsmarker ist für die Zwecke der Erfindung ein Gen, welches für ein Genprodukt kodiert, das die Selektion auf solche Zellen ermöglicht, welche den Evolutionsmarker exprimieren. In prokaryontischen Zellen eignet sich prinzipiell jedes Antibiotikaresistenzgen. Einige Beispiele sind z.B. Ampicillinrestenzgene, wie etwa das bla-Gen, welches für β-Lactamase kodiert. Weitere solcher Resistenzgene sind diejenigen, welche zum Beispiel für Aminoglycosid-3'-phosphotransferase (*npt*II) kodieren, welche Resistenz gegen Kanamycin und G418 vermitteln, sowie Chloramphinicolacetyltransferase (*cat*)*.* Für ein prokaryontisches System ist als Evolutionsmarker cat bevorzugt.

In Hefesystemen können als Evolutionsmarker folgende Gene verwendet werden, z.B. *his3, trp1, ura3* oder *leu2.* In prokaryontischen und HefeSystemen bietet sich hier z.B. das Selektionsgen *his3* an, welches Histidin-Prototrophie vermittelt und dessen Dosiseffekt über die Konzentration von 3-Aminotriazol im Medium eingestellt werden kann. Auch in anderen Fällen, wie vorzugsweise Säugerzellen, kann man ähnliche Resistenzgene als Evolutionsmarker verwenden.

Die Expression des Evolutionsmarkers erlaubt eine Selektion zum Beispiel dahingehend, dass nur jene Zellen selektioniert werden, welche sich schneller vermehren als diejenigen Zellen, welche den Evolutionsmarker nicht oder schlechter exprimieren (sofern es sich bei dem Evolutionsmarker um ein Resistenzgen handelt), oder Zellen, welche den Expressionsmarker weniger gut oder gar nicht exprimieren (falls es sich bei dem Evolutionsmarker um ein für die Zelle nachteiliges Gen handelt).

Somit steht die Wachstumsgeschwindigkeit einer Zelle in direktem Zusammenhang mit der Expression des Selektionsgens.

Es ist weiterhin möglich, auch herkömmliche Selektionsmarker als Evolutionsmarker zu verwenden. Diese Evolutionsmarker können beispielsweise für Antibiotikaresistenzgene kodieren oder dgl.

Eine weitere Abwandlung des Selektionssystems besteht darin, dass man als Evolutionsmarker ein Gen verwendet, welches für ein Protein kodiert, das auf der Zelloberfläche exprimiert wird. Es ist dann möglich, Zellen, die dieses Protein exprimieren, aufgrund der Bindung des exprimierten Proteins an einen entsprechenden Bindepartner, der an eine feste Matrix gekoppelt ist, an diese feste Matrix zu binden. Dies ermöglicht dann eine Selektion der den Evolutionsmarker exprimierenden Zellen gegenüber den übrigen Zellen, welche nicht zur Bindung an die feste Matrix in der Lage sind.

Der Evolutionsmarker befindet sich unter der Kontrolle einer modulierbaren Expressionskontrollsequenz. Hierfür sind alle Arten von *in trans* und/oder *in cis* aktivierbaren Promotoren, Enhancern und ähnlichen Aktivatorsequenzen geeignet. In einer bevorzugten Ausführungsform handelt es sich bei der Expressionskontrollsequenz um eine Sequenz, die eine Sequenz beinhaltet, welche zur Aktivierung die Bindung eines Proteins benötigt. Ein Beispiel ist eine "upstream activating sequence" (UAS).

Vorzugsweise wird für eine derartige Modulation des Evolutionsmarkers das Prinzip des Two-Hybrid- Systems angewendet. Dieses System ist im Stand der Technik wohl bekannt und dient zur Detektion von Protein-Protein-Interaktionen. Das Two-Hybrid-System beruht auf einem Komplex aus zwei Fusionsproteinen, wobei das eine Fusionsprotein eine Bindedomäne für die in der Expressionskontrollsequenz befindlichen Aktivierungssequenz (UAS), fusioniert mit oder gekoppelt an ein Protein X, aufweist. Der zweite Proteinkomplex umfasst ein zum untersuchendes. Protein Y und eine Aktivatordomäne, welche mit der für die Transkription verantwortlichen Polymerase wechselwirkt. Nur wenn eine Bindung zwischen X und Y stattfindet, kann die Expressionskontrollsequenz aktiviert und das davon kontrollierte Gen, d.h. also das Selektionsgen, exprimiert werden.

Für den Fachmann ist klar, dass dieses System der beiden Bindepartner X und Y bzw. Y' sehr gut für das Verfahren der vorliegenden Erfindung angewandt werden kann. Ein großer Nachteil des Yeast-Two-Hybrid-Systems ist jedoch, dass es lediglich zum Nachweis von Protein-Protein-Interaktionen zwischen zwei bekannten Proteinen oder zum Screenen von bereits generierten Proteinvarianten geeignet ist. Das im Stand der Technik bekannte Two-Hybrid-System weist keinen Schritt des Evolvierens von Proteinen auf.

Das erfindungsgemäße Verfahren hingegen erlaubt die Generierung von Varianten Y' des Proteins Y in dem bereitgestellten System, d.h. innerhalb der Zelle, von selbst. Es ist somit möglich, zu variierende Proteine Y sich selbst in eine bestimmte Richtung entwickeln zu lassen, die man durch die Bindefähigkeit der Varianten Y' an das Targetmolekül X steuern kann.

Wenn im System Varianten von Y generiert werden, welche eine höhere Affinität für X aufweisen bzw. in Gegensatz zu Y überhaupt erst eine Affinität für X ausweisen, so erlaubt die Expression des Selektionsgens das Überleben bzw. die schnellere Vermehrung und somit die Selektion derjenigen Zellen, die eine Variante Y' des variierenden Proteins mit verbesserten Eigenschaften aufweisen.

Im bekannten Yeast-Two-Hybrid-System wird zur Aktivierung der Expressionskontrollsequenz das Protein Gal4 verwendet, welches eine Bindedomäne für eine Upstream-Aktivierungssequenz aufweist, sowie eine Aktivierungsdomäne, welche mit der Polymerase wechselwirkt. Es ist möglich, diese beiden Domänen des Gal4-Proteins zu trennen und jede Domäne jeweils mit einem weiteren Molekül entweder als Fusionsprotein bereit zu stellen oder auf andere Art und Weise zu koppeln, wobei dann die Wechselwirkung zwischen den weiteren Molekülen, z.B. X und Y bzw. Y', es ermöglicht, die beiden Gal4-Domänen wieder in räumliche Nähe zueinander zu bringen, was dann die Modulation der Expressionskontrollsequenz ermöglicht.

Die zweite und dritte Nukleinsäure können entweder als separate Nukleinsäuren vorliegen, z.B. in Form von Plasmiden, oder sie können auch beide auf demselben Plasmid vorhanden sein. Die erste Nukleinsäure sollte, wie oben bereits erwähnt, eine replizierbare Nukleinsäure darstellen, vorzugsweise ein RNA-Replikon, welches von RNA-Replikasen repliziert werden kann. Es ist allerdings auch möglich, die erste Nukleinsäure in Form eines Plasmids oder einer anderen Form einer transfizierbaren oder transformierbaren Nukleinsäure bereitzustellen, wobei diese Nukleinsäure dann für eine entsprechend replizierbare erste Nukleinsäure kodieren sollte.

Für die Zwecke der vorliegenden Erfindung ist es möglich, das bekannte Yeast-Two-Hybrid-System oder ein entsprechendes System zu verwenden, wobei die Wechselwirkung, d.h. die Bindung des Proteins Y und/oder Varianten Y' des Proteins Y an ein Targetmolekül X, die Modulation der modulierbaren Expressionskontrollsequenz und somit die Modulation der Expression des Evolutionsmarkers ermöglichen.

Vorzugsweise werden somit zwei Expressionsmodulatoren verwendet, wobei die Wechselwirkung von beiden Expressionsmodulatoren miteinander, entweder direkt oder indirekt, über X und Y nzw. Y' für die Modulation der Expressionskontrollsequenz des Evolutionsmarkers notwendig ist.

In einer bevorzugten Ausführungsform ist das zu variierende Protein Y, welches von der ersten Nukleinsäure, insbesondere einem RNA-Replikon kodiert wird, mit entweder dem ersten oder dem zweiten Expressionsmodulator gekoppelt. Dabei handelt es sich entweder um ein Fusionsprotein zwischen Y und dem ersten Expressionsmodulator oder um ein Fusionsprotein zwischen Y und dem zweiten Expressionsmodulator oder das Protein Y wird auf andere Art und Weise, z.B. über Biotin-Streptavidin-Bindung mit einem der beiden Expressionsmodulatoren gekoppelt.

Das Targetmolekül X kann ein Protein sein oder auch ein anderes Molekül, z.B. eine Nukleinsäure. Wichtig ist, dass X entweder mit einem Expressionsmodulator gekoppelt ist oder mit diesem ein Fusionsprotein bildet oder selbst den entsprechenden Expressionsmodulator darstellt.

In einer Ausführungsform ist z.B. der erste (oder alternativ der zweite) Expressionsmodulator ein Molekül, welches an eine Upstream-Aktivierungssequenz (UAS) bindet. Der zweite (oder alternativ der erste) Expressionsmodulator moduliert die Expression durch Bindung an die entsprechende Polymerase, wobei eine Modulation der Expression nur dann erfolgt; wenn gleichzeitig der erste (oder alternativ der zweite) Expressionsaktivator mit der UAS wechselwirkt und der zweite (oder alternativ der erste) Expressionsaktivator mit der Polymerase wechselwirkt. Mit diesen Expressionsmodulator gekoppelt oder mit diesen fusioniert sind jeweils die Moleküle X und Y bzw. Y', wobei somit die Wechselwirkung zwischen X und Y bzw. zwischen X und Y' es ermöglicht, dass die beiden Expressionsmodulatoren die Expression modulieren können.

Vorzugsweise wird in der vorliegenden Erfindung die modulierbare Expressionskontrollsequenz aktiviert, wodurch der Evolutionsmarker exprimiert wird und somit der Zelle einen Wachstumsvorteil verschafft. Es ist aber auch möglich, ein System zu verwenden, bei dem die Modulation eine Inhibierung ist.

In einer bevorzugten Ausführungsform, welche sich das Two-Hybrid-System zu nutze macht, werden das Protein X und das Protein Y jeweils als Fusionsproteine mit der DNA-Bindedomäne (vorzugsweise als X/Bindedomäne) und mit der Aktivatordomäne (vorzugsweise Y/Aktivatordomäne) bereitgestellt. Es ist jedoch auch möglich, anstelle von Fusionsproteinen die gewünschten Einheiten über weitere Moleküle aneinander zu koppeln, beispielsweise über Linker oder Streptavidin/Biotin-Bindungen oder dgl.

Anstelle des herkömmlichen Two-Hybrid-Systems können auch Tri-Hybrid-Systeme verwendet werden oder es können Proteinkomplexe mit mehr Untereinheiten verwendet werden.

Über das One-Hybrid-System sind weiterhin Proteine Y evolvierbar, die an eine gegebene DNA-Sequenz binden.

Die experimentelle Vorgehensweise bei einer bevorzugten Ausführungsform der Erfindung erfolgt zunächst über die Transformation der ersten Nukleinsäure in einen Hefe oder *E*. *coli* Stamm, der bereits beide anderen Nukleinsäuren enthält. Der Transformationsansatz wird dann unter Evolutionsmarker-Selektionsbedingungen in Flüssigkultur angezogen, wobei die Wachstumsgeschwindigkeit der Kultur ständig überwacht wird. Bevor die Kultur in die logarithmische Phase übergeht, wird ein Aliquot dieser in frisches Medium überimpft, und das weitere Wachstum beobachtet. Das Überimpfen kann dabei auch automatisch, oder über eine kontinuierliche Medienzugabe und Kulturentnahme erfolgen.

Ein Endpunkt des Evolutionsprozesses ist erreicht, wenn auch trotz Erhöhung des Selektionsdrucks keine weitere Steigerung der Wachstumsgeschwindigkeit beobachtet wird. Die Isolierung und Sequenzierung der Replikonsequenz(en) einzelner Kolonien identifiziert potentielle Kandidaten, die in der Folge individuell auf die Affinitäten ihrer Genprodukte untersucht werden. Ebenso ist es möglich, die Gesamtheit aller Varianten, die zum Endpunkt innerhalb des Systems vorliegen, als Genbank zu behandeln, und die wiederum besten Varianten über einen Selektionsassay (z.B. Phage Display) zu identifizieren.

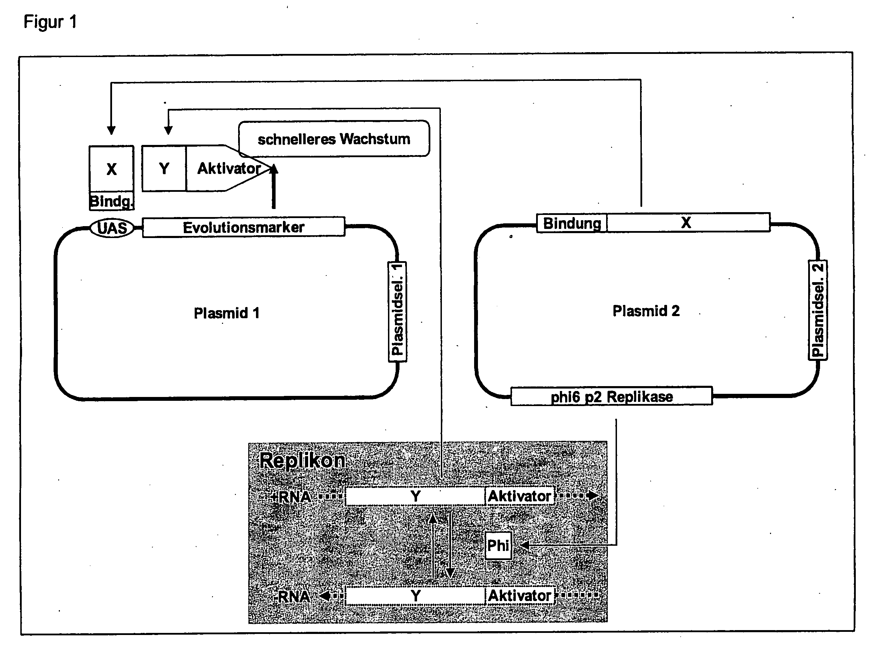

Abbildung 1 zeigt eine schematische Darstellung einer bevorzugten Ausführungsform des Systems der autonomen Evolution *in vivo,* welches unten näher beschrieben ist.

Ein weiterer Gegenstand der vorliegenden Erfindung ist eine Zelle, umfassend
(i) eine erste Nukleinsäure, die für ein zu variierendes Protein Y kodiert,
(ii) eine zweite Nukleinsäure, die für ein Targetmolekül X kodiert, wobei Y in der Lage ist, an das Targetmolekül X zu binden,
(iii) eine dritte Nukleinsäure, welche für einen Evolutionsmarker unter der Kontrolle einer modulierbaren Expressionskontrollsequenz kodiert, und, die Bildung von Varianten Y' des Proteins Y erfolgt, wobei die Varianten Y' des Proteins Y durch veränderte Bindungseigenschaften an das Targetmolekül X gekennzeichnet sind.

Die Zelle kann mit den entsprechenden Nukleinsäuren transformiert oder transfiziert sein, wobei herkömmliche und dem Fachmann wohl bekannte Methoden zur Transformation und Transfektion verwendet werden können.

Ein noch weiterer Gegenstand der vorliegenden Erfindung ist ein Kit zur Durchführung des erfindungsgemäßen Verfahrens, d.h. zur Entwicklung von Varianten Y' eines beliebigen Proteins Y, wobei die Varianten Y' des Proteins Y durch veränderte Bindungseigenschaften an das Targetmolekül X gekennzeichnet sind,
(i) eine Zelle,
(ii) eine erste Nukleinsäure, die für ein zu variierendes Protein Y kodiert,
(iii) eine zweite Nukleinsäure, die für ein Targetmolekül X kodiert, wobei Y in der Lage ist, an das Targetmolekül X zu binden,
(iv) eine dritte Nukleinsäure, welche für einen Evolutionsmarker unter der Kontrolle einer modulierbaren Expressionskontrollsequenz kodiert, und, wobei, die Bildung von Varianten Y' des Proteins Y erfolgt, wobei die Varianten Y' des Proteins Y durch veränderte Bindungseigenschaften an das Targetmolekül X gekennzeichnet sind, sowie
(v) gegebenenfalls ein geeignetes Selektionsmedium.

Das hierin offenbarte Verfahren hat den Vorteil, dass es aufwändiges Screening-Verfahren zu einem großen Teil vermeidet und dass beliebige Proteine evolviert werden können.

Insbesondere durch die Kombination der zielgerichteten Evolution mit einem System, welches auf dem Yeast-Two-Hybrid-System beruht, ist es möglich, die Evolution von Proteinen mit verbesserten Bindungseigenschaften für ein Targetmolekül X autonom zu erreichen. Es ist hierbei nicht mehr notwendig, nach jeder Generation die gebildeten Varianten zu isolieren und zu charakterisieren, sondern das System kann über mehrere Generationen sich selbst überlassen werden, bis sich die Varianten Y' des Proteins Y mit den besten Bindungseigenschaften für das entsprechend ausgewählte Targetmolekül X von selbst gebildet haben.

Um über die RNA-Replikon gestützte *in vivo* Evolution auch die Verbesserung von extrazellulären Proteinen (z.B. Immunglobuline, Anticaline, etc.), Rezeptoren etc. zu ermöglichen, kann das Zielmolekül X in der Zelle so exprimiert werden, dass es zur Zelloberfläche bzw. ins Periplasma der Zelle exportiert wird und in diesem Kompartiment verankert bleibt. Die replikon-codierten Interaktionsvarianten werden ebenfalls auf der Zelloberfläche verankert und können gegebenenfalls über eine flexible Linker-Domäne mit dem Zielmolekül X interagieren. Eine erhöhte Interaktion führt ihrerseits (eventuell über ein drittes Molekül, welches von der Interaktion verdrängt wird) zu einem Selektionsvorteil der betreffenden Zelle. Das Signal kann z.B. über eine Konformationsänderung der Membranverankernden Domäne mit darauffolgender Signalkaskade in den Zellkern erfolgen (z.B. Optimierung der Affinität von Insulin an den Insulin-Rezeptor.)

Denkbar ist auch die Regulation von Membrantransportern, die entweder essentielle Moleküle ins Zellinnere oder potentielle Zellgifte aus der Zelle befördern.

Denkbar ist auch die Expression von Targetmolekül X als di-, tri- oder multimeres Protein, wodurch es bei einer zunehmenden Affinität zwischen den Molekülen zu einer Vernetzung derselben und einer Clusterbildung auf der Oberfläche kommt. Dies kann Auswirkungen auf die Adhäsion der Zelle an Oberflächen haben, wodurch wiederum eine Selektion stark adhärierender Zellen möglich ist, während weniger stark haftende Zellen mit zunehmender Stringenz eliminiert werden.

Ebenfalls über Adhäsion erfolgt die Selektion von replikon-codierten Interaktionsvarianten Y', die in Zellen an der Zelloberfläche exprimiert werden auf folgendem Wege. Das Targetmolekül X (Protein, Peptid, Nukleinsäure, andere Polymere, kleine organische oder anorganische Moleküle (z.B. Zwischenzustand einer enzymatisch katalysierten Reaktion)) wird dabei an eine feste oder flüssige Matrix (oder Micellen) gebunden und in Kontakt mit der Zellsuspension gebracht. Zellen, die höheraffine Varianten exprimieren haben eine höhere Affinität zu der festen Matrix. Mit zunehmender Stringenz (z.B. Salz, pH, Bindungskompetitoren etc.) werden schwach adhärierende Zellen weggewaschen, die verbleibenden Zellen werden frischem Medium ausgesetzt und neue Matrix zur Verfügung gestellt, während ein Teil der alten Matrix entfernt wird. Dieser Prozess ist leicht automatisierbar.

Zellen, welche sehr gute Binder exprimieren, werden sich von der Oberfläche nicht mehr lösen. Dennoch sind sie gegebenenfalls noch zur Zellteilung fähig. Tochterzellen haben bei besetzter unmittelbarer Umgebung die Gelegenheit, entfernte Bereiche mit frischer Matrix zu erreichen. Von Schritt zu Schritt (wobei ein "Schritt" definiert ist vom animpfen neuen Mediums bis zum nächsten Animpfen mit einem Teil der Matrix) kann dabei der Matrixtyp gewechselt werden. Also z.B. Magnetische Beads und Polyvinyl-Oberflächen (Eliza plate). Dadurch wird von einem Schritt zum nächsten immer nur "neu besetzte Matrix" überführt.

In jedem Fall (intra- wie extrazellulär) besteht die Möglichkeit, die Affinität nicht nur zu Proteinen hin zu erhöhen, sondern auch zu kleinen organischen oder anorganischen Molekülen. Hierzu kann das Molekül beispielsweise kovalent an Biotin gekoppelt sein. Dieses Molekül wird in das Cytoplasma bzw. ins extrazelluläre Medium eingebracht (evtl. ist es membrangängig). Anstelle des Zielmoleküls X wird in diesem Fall Streptavidin (als Fusion im Falle von Two-Hybrid) exprimiert. Die Zielmolekül/Biotin-Chimäre bindet an Streptavidin und präsentiert somit das Zielmolekül um als Affinitätstarget zu fungieren.

### Beschreibung der Abbildung

### Abbildung 1: Schematische Darstellung des Systems zur autonomen Evolution in vivo.

Plasmid 1 codiert unter der Kontrolle der upstream activating sequence (UAS) für einen Evolutionsmarker, welcher schnelleres Wachstum vermittelt. Das Fusionsprotein aus DNA-Bindedomäne und der Komponente X (Bindung/X) wird von Plasmid 2 codiert und bindet an die UAS. Abhängig von der Stärke der Interaktion zwischen der Komponente X und dem Protein Y bzw. Y' kann die fusionierte Aktivierungsdomäne (Aktivator) den zelleigenen Transkriptionskomplex rekrutieren und es kommt, zu einer abhängigen Expression des Evolutionsmarkers. Das Fusionsprotein Y/Aktivator wird von einem Replikon codiert, welches autonom und fehlerbehaftet von einer RNA-abhängigen RNA-Polymerase (Phi) repliziert wird.

### Beispiel

Ausgehend von einem synthetischen Gen wird ein PCR-Produkt generiert, welches folgende Elemente enthält: T7-Promoter + Leserahmen für die Aktivierungsdomäne des Hefeproteins Gal4 (Fields und Sternglanz, 1994) in Fusion mit einem Peptid von 30 zufälligen Aminosäuren (= Act/30X). Durch die Zufälligkeit der 30 C-terminalen Aminosäuren wurde eine Genbank erzeugt, die eine Anzahl von ca. 10⁹ unterschiedlichen Varianten enthielt. In einer Transkriptionsreaktion wurden 5 pmol des PCR Produkts mit T7-Polymerase in einer 100 µl Reaktion transkribiert (= Act/30X Replikon).

In das Plasmid pAS (Fields und Sternglanz, 1994) wird eine Hefe-Expressionskassette für die RNA-abhängige RNA-Polymerase Qβ (Acc. No. AAM33128) kloniert (= pAS-Qβ). Dieses Plasmid erlaubt eine Selektion in Hefe auf Tryptophan-Prototrophie.

Der Leserahmen für HIV-Integrase (Acc. No. AAC61700.1) wird in Fusion mit der Gal-DNA-Bindedomäne in das Plasmid pAS-Qβ kloniert (=pAS-Qβ-Int), in den Hefestamm Y190 transformiert und auf Tryptophan-Prototrophie selektioniert.

Die mit pAS-Qβ-Int transformierte Hefekultur wird mit 10 µg Act/30X Replikon RNA transformiert (Transformationsrate ca. 500.000) und direkt in 50 ml Tryptophan/Histidin-dropout Flüssigkultur unter Zugabe von 12 mM 3-Aminotriazol (3-AT) bei 30 °C unter Schütteln inkubiert.

Nach Erreichen einer OD (600 nm) von 3,0 werden 50 ml frisches Medium mit 200 µl der Kultur angeimpft und erneut bis zum Erreichen der OD von 3,0 geschüttelt (dies entspricht einer Generationszahl von 8 Verdoppelungen). Die Zeit zum Erreichen dieser OD dividiert durch acht entspricht der Generationszeit der Hefepopulation.

Nähert sich die Generationszeit 60 min, wird die 3-AT Konzentration zunächst auf 25 mM (nach der 5. Überimpfung), dann auf 50 mM (nach der 8. Überimpfung) erhöht. Nach der 10. Überimpfung ist eine Generationszeit von 85 min erreicht, die auch nach weiterer Überimpfung nicht weiter sinkt.

20 µl der Hefekultur der 10. Überimpfungskultur werden auf Tryptophan/Histidin-dropout Platten ausplattiert und 3 Tage bei 30 °C inkubiert. Die größte Kolonie wird in 30 ml Tryptophan/Histidin-dropout Medium überimpft, zu einer OD von 3,0 angewachsen und abzentrifugiert. Das Zellpellet wird mit Glasperlen in STET Puffer, Phenol und Chloroform aufgeschlossen, der wässrige Überstand 2x mit Chloroform ausgeschüttelt und enthaltene Nukleinsäuren mit Ethanol gefällt. Das getrocknete Pellet (DNA & RNA) wird in 100 µl 10 mM Tris resuspendiert. 5 µl davon werden mit einem für das Replikon spezifischen Oligonukleotid mit Reverser Transkriptase in DNA umgeschrieben und über PCR amplifiziert. Das PCR Produkt wird in einen geeigneten Vektor subkloniert und in *E. coli* transformiert.

Die Sequenzauswertung von 384 *E. coli* Klonen ergibt eine statistische Verteilung unterschiedlicher Sequenzen, welches für evolvierte Act/30X Varianten kodieren, die eine erhöhte Affinität zur HIV Integrase aufweisen. Nähere Untersuchungen zeigen wiederkehrende Aminosäuremotive innerhalb des variablen Peptids (30X), welche für die erhöhte Affinität verantwortlich sind.

## Patentansprüche

1. Verfahren zur Herstellung von Varianten Y' eines Proteins Y, wobei die Variante Y' des Proteins Y durch veränderte Bindungseigenschaften an ein Targetmolekül X **gekennzeichnet** sind, umfassend die Schritte:
(a) Bereitstellen einer Zelle, enthaltend
(a1) eine erste Nukleinsäure, die für ein zu variierendes Protein Y kodiert,
(a2) eine zweite Nukleinsäure, die für das Targetmolekül X kodiert, und
(a3) eine dritte Nukleinsäure, welche für einen Evolutionsmarker unter der Kontrolle einer modulierbaren Expressionskontrollsequenz kodiert,
wobei die Expression des Evolutionsmarkers durch die Bindung von Y und/oder Y' an X moduliert wird,
(b) Kultivieren der Zelle unter Bedingungen, so dass
(b1) eine Bildung von Varianten Y' des Proteins Y auf Nukleinsäureebene erfolgt und
(b2) eine Selektion auf diejenigen Zellen erfolgt, welche eine gegenüber der Zelle aus (a) modulierte Expression des Evolutionsmarkers aufweisen, und
(c) Identifizieren und gegebenenfalls Isolieren derjenigen Zellen, die eine modulierte Expression des Evolutionsmarkers aufweisen und
(d) gegebenenfalls Identifizieren und/oder Charakterisieren von Varianten Y' des Proteins Y in den Zellen aus (c).

2. Verfahren nach Anspruch 1,
wobei die Zelle ein prokaryontische oder eukaryontische Zelle ist.

3. Verfahren nach Anspruch 2,
wobei die Zelle ausgewählt wird aus *E*. *coli,* Hefezellen, Pflanzenzellen und Vertebratenzellen, insbesondere Säugerzellen.

4. Verfahren nach einem der vorhergehenden Ansprüche,
wobei die erste Nukleinsäure mit einer gegenüber der natürlichen Variationsrate erhöhten Variationsrate repliziert und/oder transkribiert wird.

5. Verfahren nach einem der vorhergehenden Ansprüche,
wobei die erste Nukleinsäure in Form eines RNA-Replikons vorliegt und die Zelle aus (a) zusätzlich eine Replikase enthält, die in der Lage ist, das RNA-Replikon unter Bildung von Varianten zu replizieren.

6. Verfahren nach Anspruch 5,
wobei das RNA-Replikon ausgewählt ist aus linearen RNA-Sequenzen, Genomen aus RNA-basierenden Organismen, RNA-Plasmiden, RNA-Bakteriophagen und RNA-Analoga.

7. Verfahren nach Anspruch 5 oder 6,
wobei die Replikase ausgewählt ist aus RNA-abhängigen RNA-Polymerasen, wie etwa Qβ-, Phi6-, Phi8-, Phi9-, Phi10-,Phi 11-, Phi13- und Phi14-Replikasen.

8. Verfahren nach einem der vorhergehenden Ansprüche,
wobei der Evolutionsmarker ausgewählt wird aus Genen, deren Expression eine schnellere Vermehrung der Zelle ermöglicht.

9. Verfahren nach einem der vorhergehenden Ansprüche,
wobei der Evolutionsmarker ausgewählt wird aus Genen, die für oberflächenständige Proteine kodieren, welche die Bindung der Zelle an eine feste Matrix ermöglichen.

10. Verfahren nach einem der vorhergehenden Ansprüche,
wobei das Verfahren intrazellulär durchgeführt wird.

11. Verfahren nach einem der vorhergehenden Ansprüche,
wobei das Protein Y und dessen Varianten Y' jeweils an einen ersten Expressionsmodulator gekoppelt sind und X an einen zweiten Expressionsmodulator gekoppelt ist, wobei für die Modulation der Expression des Evolutionsmarkers eine Wechselwirkung des ersten Expressionsmodulators und des zweiten Expressionsmodulators mit der für den Evolutionsmarker kodierenden Nukleinsäure notwendig ist.

12. Verfahren nach einem der vorhergehenden Ansprüche,
wobei X ein Protein ist.

13. Verfahren nach Anspruch 11 oder 12,
wobei die erste Nukleinsäure für ein Fusionsprotein aus Y und dem ersten Expressionsmodulator kodiert und die zweite Nukleinsäure für ein Fusionsprotein aus X und dem zweiten Expressionsmodulator kodiert, oder
wobei die erste Nukleinsäure für ein Fusionsprotein aus Y und dem zweiten Expressionsmodulator kodiert und die zweite Nukleinsäure für ein Fusionsprotein aus X und dem ersten Expressionsmodulator kodiert.

14. Verfahren nach einem der Ansprüche 11 bis 13,
wobei der erste Expressionsmodulator ein Modulator für eine Polymerase ist.

15. Verfahren nach einem der Ansprüche 11 bis 13,
wobei der zweite Expressionsmodulator ein Modulator für eine Polymerase ist.

16. Verfahren nach Anspruch 14,
wobei die modulierbare Expressionskontrollsequenz auf der dritten Nukleinsäure eine Upstream-Aktivierungssequenz (UAS) umfasst und der zweite Expressionsmodulator ein Bindeprotein ist, welches in der Lage ist, spezifisch an die UAS zu binden.

17. Verfahren nach Anspruch 15,
wobei die modulierbare Expressionskontrollsequenz auf der dritten Nukleinsäure eine Upstream-Aktivierungssequenz (UAS) umfasst und der erste Expressionsmodulator ein Bindeprotein ist, welches in der Lage ist, spezifisch an die UAS zu binden.

18. Verfahren nach einem der vorhergehenden Ansprüche,
wobei die modulierbare Expressionskontrollsequenz eine Upstream-Aktivierungssequenz (UAS) umfasst, welche eine Bindung des Proteins Gal4 erlaubt.

19. Verfahren nach einem der vorhergehenden Ansprüche,
wobei die Bindung von Y und/oder Y' an X eine Aktivierung der modulierbaren Expressionskontrollsequenz auf der dritten Nukleinsäure ermöglicht.

20. Zelle, umfassend
(i) eine erste Nukleinsäure, die für ein zu variierendes Protein Y kodiert,
(ii) eine zweite Nukleinsäure, die für ein Targetmolekül X kodiert, wobei Y in der. Lage ist, an das Targetmolekül X zu binden,
(iii) eine dritte Nukleinsäure, welche für einen Evolutionsmarker unter der Kontrolle einer modulierbaren Expressionskontrollsequenz kodiert, und die Bildung von Varianten Y' des Proteins Y erfolgt,
wobei die Varianten Y' des Proteins Y durch veränderte Bindungseigenschaften an das Targetmolekül X gekennzeichnet sind.

21. Zelle nach Anspruch 20,
wobei die erste Nukleinsäure in Form eines RNA-Replikons vorliegt und die Zelle zusätzlich eine Replikase enthält, die in der Lage ist, das RNA-Replikon unter Bildung von Varianten zu replizieren.

22. Zelle nach Anspruch 20 oder 21,
wobei es sich um eine prokaryontische oder eukaryontische Zelle handelt.

23. Kit zur Herstellung von Varianten Y' eines Proteins Y, wobei die Varianten Y' des Proteins Y durch veränderte Bindungseigenschaften an das Targetmolekül X gekennzeichnet sind, umfassend
(i) eine Zelle,
(ii) eine erste Nukleinsäure, die für ein zu variierendes Protein Y kodiert,
(iii) eine zweite Nukleinsäure, die für ein Targetmolekül X kodiert, wobei Y in der Lage ist, an das Targetmolekül X zu binden,
(iv) eine dritte Nukleinsäure, welche für einen Evolutionsmarker unter der Kontrolle einer modulierbaren Expressionskontrollsequenz kodiert, und
wobei die Bildung von Varianten Y' des Proteins Y erfolgt, wobei die Varianten Y' des Proteins Y durch veränderte Bindungseigenschaften an das Targetmolekül X gekennzeichnet sind, sowie
(v) gegebenenfalls ein geeignetes Selektionsmedium.

24. Kit zur Herstellung von Varianten Y' eines Proteins Y, umfassend:
(i) eine Zelle, wie in einem der vorhergehenden Ansprüche 20 bis 22 definiert,
(ii) Selektionsmedium, mit dem die Zellen aufgrund der modulierten Expression des Selektionsgens selektioniert werden können.

## Claims

1. Method for producing variants Y' of a protein Y, where the variants Y' of the protein Y are **characterized by** modified binding properties to a target molecule X, comprising the steps:
(a) providing a cell containing
(a1) a first nucleic acid which codes for a protein Y to be varied,
(a2) a second nucleic acid which codes for the target molecule X, and
(a3) a third nucleic acid which codes for an evolution marker under the control of an expression control sequence that can be modulated,
wherein the expression of the evolution marker is modulated by
the binding of Y and/or Y' to X,
(b) culturing the cell under such conditions that
(b1) variants Y' of the protein Y are formed at the nucleic acid level and
(b2) those cells are selected which exhibit an expression of the evolution marker that is modulated compared to the cells from (a), and
(c) identifying and optionally isolating those cells that exhibit a modulated expression of the evolution marker, and
(d) optionally identifying and/or characterizing variants Y' of the protein Y in the cells from (c).

2. Method according to claim 1,
wherein the cell is a prokaryotic or eukaryotic cell.

3. Method according to claim 2,
wherein the cell is selected from *E. coli,* yeast cells, plant cells and vertebrate cells, in particular mammalian cells.

4. Method according to one of the previous claims
wherein the first nucleic acid is replicated and/or transcribed with a variation rate that is increased compared to the natural variation rate.

5. Method according to one of the previous claims,
wherein the first nucleic acid is present in the form of an RNA replicon and the cell from (a) additionally contains a replicase that can replicate the RNA replicon to form variants.

6. Method according to claim 5,
wherein the RNA replicon is selected from linear RNA sequences, genomes from RNA-based organisms, RNA plasmids, RNA bacteriophages and RNA analogues.

7. Method according to claim 5 or 6,
wherein the replicase is selected from RNA-dependent RNA polymerases such as Qβ, phi6, phi8, phi9, phi10, phi11, phi13 and phi14 replicases.

8. Method according to one of the previous claims
wherein the evolution marker is selected from genes whose expression enables a more rapid propagation of the cell.

9. Method according to one of the previous claims
wherein the evolution marker is selected from genes which code for surface-associated proteins which enable the cell to bind to a solid matrix.

10. Method according to one of the previous claims,
wherein the method is carried out intracellularly.

11. Method according to one of the previous claims,
wherein the protein Y and its variants Y' are each coupled to a first expression modulator and X is coupled to a second expression modulator
wherein an interaction of the first expression modulator and of the second expression modulator with the nucleic acid coding for the evolution marker is necessary for modulating the expression of the evolution marker.

12. Method according to one of the previous claims,
wherein X is a protein.

13. Method according to claim 11 or 12,
wherein the first nucleic acid codes for a fusion protein consisting of Y and the first expression modulator and the second nucleic acid codes for a fusion protein consisting of X and the second expression modulator or wherein the first nucleic acid codes for a fusion protein consisting of Y and the second expression modulator and the second nucleic acid codes for a fusion protein consisting of X and the first expression modulator.

14. Method according to one of the claims 11 to 13,
wherein the first expression modulator is a modulator for a polymerase.

15. Method according to one of the claims 11 to 13,
wherein the second expression modulator is a modulator for a polymerase.

16. Method according to claim 14,
wherein the expression control sequence that can be modulated on the third nucleic acid comprises an upstream activating sequence (UAS) and the second expression modulator is a binding protein capable of specifically binding to the UAS.

17. Method according to claim 15,
wherein the expression control sequence that can be modulated on the third nucleic acid comprises an upstream activating sequence (UAS) and the first expression modulator is a binding protein capable of specifically binding to the UAS.

18. Method according to one of the previous claims,
wherein the expression control sequence that can be modulated comprises an upstream activating sequence (UAS) which allows binding of the protein Gal4.

19. Method according to one of the previous claims,
wherein the binding of Y and/or Y' to X enables activation of the expression control sequence that can be modulated on the third nucleic acid.

20. Cell comprising
(i) a first nucleic acid which codes for a protein Y to be varied,
(ii) a second nucleic acid which codes for a target molecule X wherein Y is able to bind to the target molecule X,
(iii) a third nucleic acid which codes for an evolution marker under the control of an expression control sequence that can be modulated, and variants Y' of the protein Y are formed,
wherein the variants Y' of the protein Y are **characterized by** modified binding properties to the target molecule X.

21. Cell according to claim 20,
wherein the first nucleic acid is present in the form of an RNA replicon and the cell additionally contains a replicase that can replicate the RNA replicon to form variants.

22. Cell according to claim 20 or 21,
wherein it is a prokaryotic or eukaryotic cell.

23. Kit for producing variants Y' of a protein Y,
wherein the variants Y' of the protein Y are **characterized by** modified binding properties to the target molecule X, comprising
(i) a cell,
(ii) a first nucleic acid which codes for a protein Y to be varied,
(iii) a second nucleic acid which codes for a target molecule X, wherein Y is able to bind to the target molecule X,
(iv) a third nucleic acid which codes for an evolution marker under the control of an expression control sequence that can be modulated, and
wherein variants Y' of the protein Y are formed, the variants Y' of the protein Y being **characterized by** modified binding properties to the target molecule X and
(v) where appropriate a suitable selection medium.

24. Kit for producing variants Y' of a protein Y comprising:
(i) a cell as defined in one of the previous claims 20 to 22,
(ii) selection medium with which the cells can be selected on the basis of the modulated expression of the selection gene.

## Revendications

1. Procédé de préparation de variants Y' d'une protéine Y, où les variants Y' de la protéine Y sont **caractérisés par** des propriétés modifiées de liaison à une molécule cible X, comprenant les étapes de :
(a) préparation d'une cellule, contenant
(a1) un premier acide nucléique, qui code une protéine Y à varier,
(a2) un deuxième acide nucléique, qui code la molécule cible X, et
(a3) un troisième acide nucléique, qui code un marqueur d'évolution sous le contrôle d'une séquence modulable du contrôle de l'expression, où l'expression du marqueur d'évolution est modulée par la liaison de Y et/ou Y' à X,
(b) culture de la cellule dans des conditions telles que
(b1) une formation des variants Y' de la protéine Y est réalisée au niveau des acides nucléiques, et
(b2) une sélection est réalisée pour les cellules qui présentent par rapport aux cellules de (a), une expression modulée du marqueur d'évolution,
(c) identification et le cas échéant, isolement des cellules qui présentent une expression modulée du marqueur d'évolution, et
(d) le cas échéant, identification et/ou caractérisation des variants Y' de la protéine Y dans les cellules de (c).

2. Procédé selon la revendication 1, où la cellule est une cellule procaryote ou eucaryote.

3. Procédé selon la revendication 2, où la cellule est choisie parmi *E. coli,* des cellules de levures, des cellules végétales et des cellules de vertébrés, en particulier des cellules de mammifères.

4. Procédé selon l'une quelconque des revendications précédentes, où le premier acide nucléique est répliqué et/ou transcrit avec un taux de variation augmenté par rapport au taux de variation naturel.

5. Procédé selon l'une quelconque des revendications précédentes, où le premier acide nucléique est présent sous forme d'un réplicon ARN, et la cellule de (a) contient en outre, une réplicase, qui est en mesure de répliquer le réplicon ARN avec formation de variants.

6. Procédé selon la revendication 5, où le réplicon ARN est choisi parmi les séquences linéaires d'ARN, les génomes d'organismes à ARN, les plasmides ARN, les bactériophages à ARN et les analogues d'ARN.

7. Procédé selon la revendication 5 ou 6, où la réplicase est choisie parmi les ARN-polymérase dépendant d'ARN, comme par exemple les réplicases Qβ, Phi6, Phi8, Phi9, Phi10, Phi11, Phi 13 et Phi 14.

8. Procédé selon l'une quelconque des revendications précédentes, où le marqueur d'évolution est choisi parmi des gènes dont l'expression permet une multiplication plus rapide de la cellule.

9. Procédé selon l'une quelconque des revendications précédentes, où le marqueur d'évolution est choisi parmi des gènes, qui codent une protéine de surface, qui permet la liaison des cellules sur une matrice solide.

10. Procédé selon l'une quelconque des revendications précédentes, où le procédé est réalisé de manière intracellulaire.

11. Procédé selon l'une quelconque des revendications précédentes, où la protéine Y et ses variants Y' sont couplés à un premier modulateur d'expression et X est couplé à un deuxième modulateur d'expression, où pour la modulation de l'expression du marqueur d'évolution, une interaction du premier modulateur d'expression et du deuxième modulateur d'expression avec l'acide nucléique codant le marqueur d'évolution est nécessaire.

12. Procédé selon l'une quelconque des revendications précédentes, où X est une protéine.

13. Procédé selon la revendication 11 ou 12, où le premier acide nucléique code une protéine de fusion constituée de Y et du premier modulateur d'expression et le deuxième acide nucléique code une protéine de fusion constituée de X et du deuxième modulateur d'expression, ou où le premier acide nucléique code une protéine de fusion constituée de Y et du deuxième modulateur d'expression et le deuxième acide nucléique code une protéine de fusion constituée de X et du premier modulateur d'expression.

14. Procédé selon l'une quelconque des revendications 11 à 13, où le premier modulateur d'expression est un modulateur pour une polymérase.

15. Procédé selon l'une quelconque des revendications 11 à 13, où le deuxième modulateur d'expression est un modulateur pour une polymérase.

16. Procédé selon la revendication 14, où la séquence modulable du contrôle de l'expression sur le troisième acide nucléique comprend une séquence d'activation en amont (UAS) et le deuxième modulateur d'expression est une protéine de liaison, qui est en mesure de se lier spécifiquement à l'UAS.

17. Procédé selon la revendication 15, où la séquence modulable du contrôle de l'expression sur le troisième acide nucléique comprend une séquence d'activation en amont (UAS) et le premier modulateur d'expression est une protéine de liaison, qui est en mesure de se lier spécifiquement à l'UAS.

18. Procédé selon l'une quelconque des revendications précédentes, où la séquence modulable du contrôle de l'expression comprend une séquence d'activation en amont (UAS), qui permet une liaison de la protéine Gal4.

19. Procédé selon l'une quelconque des revendications précédentes, où la liaison de Y et/ou de Y' permet une activation de la séquence modulable du contrôle de l'expression sur le troisième acide nucléique.

20. Cellule, comprenant :
(i) un premier acide nucléique, qui code une protéine Y à varier,
(ii) un deuxième acide nucléique, qui code une molécule cible X, où Y est en mesure de se lier à la molécule cible X,
(iii) un troisième acide nucléique, qui code un marqueur d'évolution sous le contrôle d'une séquence modulable du contrôle de l'expression, et où la liaison des variants Y' de la protéine Y est réalisée, où les variants Y' de la protéine Y sont **caractérisés par** des propriétés modifiées de liaison à la molécule cible X.

21. Cellule selon la revendication 20, où le premier acide nucléique est présent sous forme d'un réplicon ARN, et la cellule contient en outre, une réplicase, qui est en mesure de répliquer le réplicon ARN avec formation de variants.

22. Cellule selon la revendication 20 ou 21, où il s'agit d'une cellule procaryote ou eucaryote.

23. Kit pour la préparation de variants Y' d'une protéine Y, où les variants Y' de la protéine Y sont **caractérisés par** des propriétés modifiées de liaison à la molécule cible X, comprenant
(i) une cellule
(ii) un premier acide nucléique, qui code une protéine Y à varier,
(iii) un deuxième acide nucléique, qui code une molécule cible X, où Y est en mesure de se lier à la molécule cible X,
(iv) un troisième acide nucléique, qui code un marqueur d'évolution sous le contrôle d'une séquence modulable du contrôle de l'expression, et où la liaison des variants Y' de la protéine Y est réalisée, où les variants Y' de la protéine Y sont **caractérisés par** des propriétés modifiées de liaison à la molécule cible X, ainsi que
(v) le cas échéant, un milieu de sélection approprié.

24. Kit pour la préparation de variants Y' d'une protéine Y, comprenant :
(i) une cellule telle que définie dans l'une des revendications 20 à 22 précédentes,
(ii) un milieu de sélection, avec lequel les cellules peuvent être sélectionnées sur base de l'expression modulée du gène de sélection.
